# EUROPEAN PATENT SPECIFICATION

(11) **EP 4 411 740 B1**
(45) Date of publication and mention of the grant of the patent: **25.11.2026**
(21) Application number: 23866658.0
(22) Date of filing: 30.06.2023
(51) Int. Cl.: G16H 20/10, G16H 50/50, G16B 5/30, G16B 40/00, G16B 5/00

(54) **MODEL CONSTRUCTION METHOD, DEVICE AND MEDIUM**
VERFAHREN ZUR MODELLKONSTRUKTION, VORRICHTUNG UND MEDIUM
PROCÉDÉ DE CONSTRUCTION DE MODÈLE, DISPOSITIF ET SUPPORT

(30) Priority: 22.12.2022 CN 202211659064
(43) Date of publication of application: 07.08.2024
(73) Proprietor: Nanqi Xiance (Nanjing) High Tech Co., Ltd., Nanjing, Jiangsu 210000 (CN); Yu, Guo, Nanjing, Jiangsu 210000 (CN)
(72) Inventor: YU, Guo, Nanjing, Jiangsu 210000 (CN); YU, Yang, Nanjing, Jiangsu 210000 (CN); HAN, Luyao, Nanjing, Jiangsu 210000 (CN); ZHANG, Zhilong, Nanjing, Jiangsu 210000 (CN); LIU, Tianshuo, Nanjing, Jiangsu 210000 (CN); ZHAN, Dechuan, Nanjing, Jiangsu 210000 (CN); LI, Mingfeng, Nanjing, Jiangsu 210000 (CN)
(74) Representative: Weickmann & Weickmann PartmbB
(86) International application number: PCT/CN2023/105239
(87) International publication number: WO 2024/131026

(56) References cited:
- WO-A1-2016/161356
- CN-A- 104 267 125
- CN-A- 105 335 612
- CN-A- 115 458 175
- CN-A- 116 129 988
- VERHAEGHE JARNE ET AL: "Development and evaluation of uncertainty quantifying machine learning models to predict piperacillin plasma concentrations in critically ill patients", vol. 22, no. 1, 25 August 2022 (2022-08-25), GB, XP093269740, ISSN: 1472-6947, Retrieved from the Internet <URL:https://link.springer.com/content/pdf/10.1186/s12911-022-01970-y.pdf> [retrieved on 20250716], DOI: 10.1186/s12911-022-01970-y
- STADER FELIX ET AL: "A Comprehensive Framework for Physiologically-Based Pharmacokinetic Modeling in Matlab", 2 April 2019 (2019-04-02), XP093296717, Retrieved from the Internet <URL:https://pmc.ncbi.nlm.nih.gov/articles/PMC6657005/pdf/PSP4-8-444.pdf> [retrieved on 20250716], DOI: 10.1002/psp4.12399
- JAMES LU ET AL: "Deep learning prediction of patient response time course from early data via neural-pharmacokinetic/pharmacodynamic modeling", ARXIV.ORG, CORNELL UNIVERSITY LIBRARY, 201 OLIN LIBRARY CORNELL UNIVERSITY ITHACA, NY 14853, 22 October 2020 (2020-10-22), XP081935137, DOI: 10.1038/S42256-021-00357-4
- POYNTON MR ET AL: "Machine Learning Methods Applied to Pharmacokinetic Modelling of Remifentanil in Healthy Volunteers: A Multi-Method Comparison", JOURNAL OF INTERNATIONAL MEDICAL RESEARCH, vol. 37, no. 6, 1 November 2009 (2009-11-01), GB, pages 1680 - 1691, XP093296810, ISSN: 0300-0605, DOI: 10.1177/147323000903700603

## Description

### TECHNICAL FIELD

The present application relates to the technical field of biological information, for example, a model construction method and apparatus, a device, and a medium.

### BACKGROUND

In the clinical development stage of a drug, the pharmacokinetic and pharmacodynamic behaviors of the drug are often required to be evaluated, the behavior of the drug in a living organism mainly depends on the interaction between the drug and the organism, and numerous research methods for the quantitative structure-activity relationship and quantitative structure-effect relationship have been developed based on the drug structures. However, the prediction capability of the relevant prediction on drug in vivo behaviors is insufficient, and a "bottom-up" physiologically based pharmacokinetic (PBPK) model needs to be combined, the PBPK model combines the inherent properties of the drug and a physiological parameter of the drug, and the pharmacokinetic behaviors of the drug in the organism may be predicted based on in vitro experimental data. The PBPK model is widely applied to the drug interaction evaluation, the drug toxicity, the risk evaluation, the pharmacokinetic research and accurate medication of special people, and the like.

In the clinical application stage of the drug, to distinguish the in vivo drug exposure degrees of different patients, a large amount of clinical data are used for establishing a "top-down" group pharmacokinetic model, the temporal variation of dosage-exposure and the variation degree thereof and factors influencing the pharmacokinetic variation are quantitatively researched, and the typical characteristics and the variation of a pharmacokinetic parameter of a target group can be finally predicted. On the basis of the above, the pharmacokinetic parameter and the confidence interval thereof for unknown individuals are predicted by combining random effects and residual variations.

Regardless of the modeling means, the final goal is to predict the relationship between the drug administration dose and the efficacy/safety index, to clearly distinguish different variations brought by pharmacokinetics and pharmacodynamics, the drug in vivo concentration requires to be used as the intermediate medium. For a specific drug, the free drug concentration of the drug at different parts in the body circulation is predicted in the case of known the administration dose and the physiological parameter, and the local exposure-response (ER) relationship of the organism circulation or the target tissue is further characterized, that is, the complete model of the drug in vivo behavior is built around the dose-exposure-response relationship.

However, the "bottom-up" PBPK model covers excessive empirical formulas, model assumptions and virtual physiological parameters, and sometimes the model fails to characterize a potential relationship between the drug in vivo behavior and the drug or physiological parameter, resulting in a non-ideal model prediction result. The "top-down" group pharmacokinetic model abstracts the drug in vivo behavior as the pharmacokinetic parameter, ignores the deep rule causing the drug in vivo behavior difference, and has poor generalization for the unseen sample.

In recent years, the machine learning method has been widely applied to practical problems of multiple disciplines, and mechanistic prior knowledge and the black box model are gradually fused. While the machine learning strong predictive capability is maintained, the prior knowledge is added to form the semi-mechanistic model, whereby the interpretability of the model is improved, however, the drug in vivo behavior cannot be evaluated accurately yet.

Verhaeghe et al. (BMC MEDICAL INFORMATICS AND DECISION MAKING VOLUME, vol. 22, no. 1, 2022) relates a development and evaluation of uncertainty quantifying machine learning models to predict piperacillin plasma concentrations in critically ill patients.

### SUMMARY

The present application provides a model construction method, a device, and a medium, a semi-mechanistic model is established by combining a machine learning method with physiological prior knowledge, anatomical prior knowledge, and the like so that a model training process based on data driving can be achieved, and a local drug in vivo behavior and an overall drug in vivo behavior can be accurately predicted.

The invention is set out in the appended set of claims.

### BRIEF DESCRIPTION OF DRAWINGS

To more clearly explain the technical schemes in embodiments of the present application, the drawings used for describing the embodiments will be briefly introduced below.
FIG. 1 is a flowchart of a model construction method according to an embodiment of the present application;
FIG. 2 is a diagram illustrating a configuration of a physiological model structure according to an embodiment of the present application;
FIG. 3 is a flowchart of another model construction method according to an embodiment of the present application;
FIG. 4 is a flowchart of another model construction method according to an embodiment of the present application;
FIG. 5 is a schematic structural diagram of a model construction apparatus according to an embodiment of the present application; and
FIG. 6 is a structural block diagram of an electronic device according to an embodiment of the present application.

### DETAILED DESCRIPTION

The technical schemes in embodiments of the present application will be described below in conjunction with the drawings in the embodiments of the present application.

The terms "first", "second", "original", "middle", "candidate", and "target" in the specification and claims of the present application and the foregoing accompanying drawings are used to distinguish between similar objects, and do not need to be used to describe a specific order or sequence. **In** addition, the terms "include" and "have" and any modifications thereof are intended to cover non-exclusive inclusion, for example, processes, methods, systems, products or devices that contain a series of steps or units are not necessarily limited to those steps or units that are clearly listed, but may include other steps or units that are not clearly listed or are inherent to these processes, methods, products or devices.

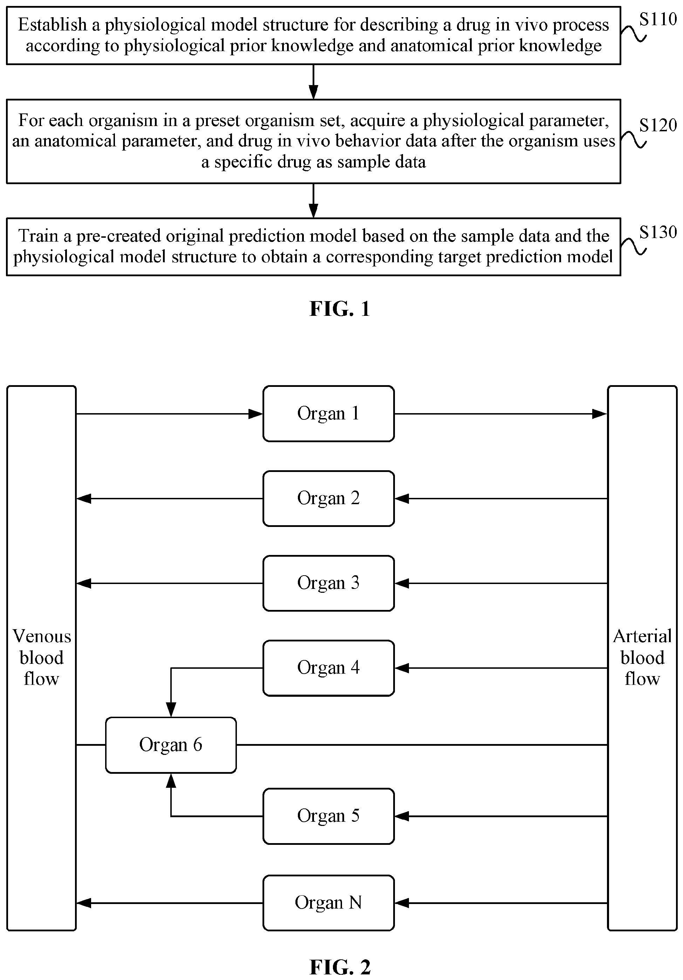

There are many machine learning methods for performing end-to-end prediction on the in vitro property or the in vivo behavior of a drug. FIG. 1 is a flowchart of a model construction method according to an embodiment of the present application. This embodiment may be applicable to the case in which a target prediction model is constructed by using prior knowledge. The method is computer implemented and performed by an electronic device. The method may be performed by a model construction apparatus. The model construction apparatus may be implemented in a form of hardware and/or software. The model construction apparatus may be configured in an electronic device. As shown in FIG. 1, the method includes the following.

In S110, a physiological model structure for describing a drug in vivo process is established according to physiological prior knowledge and anatomical prior knowledge.

The physiological model structure is used for characterizing an association of a local interaction between the drug and an organism with known physiological characteristics, anatomical characteristics and drug characteristics. In an actual operation process, the physiological prior knowledge may be represented by using physiological characteristics of different organisms, and the anatomical prior knowledge may be represented by using anatomical characteristics of different organisms. Different organisms may include human and model animals commonly used in the field of medical research and development, and the model animals include, but are not limited to, rodents, non-human primates, canines, rabbits, pigs, zebrafishes, and other animal species. The physiological characteristics may include, but are not limited to, a metabolic characteristic and a growth and development characteristic of the organism. The anatomical characteristics may include, but are not limited to, structural and dynamic change characteristics of organ tissue. The physiological characteristics and the anatomical characteristics of different organisms may be obtained from an existing database. Exemplarily, in a case of the organism being a human body, typical physiological and anatomical characteristics of different age groups and different race populations may be acquired from a certain publication. Alternatively, physiological and anatomical characteristics of multiple mode organisms are acquired from other publications.

The local interaction between the drug and the organism includes an action of the organism on the drug and an action of the drug on the organism. The action of the organism on the drug is pharmacokinetic (PK) research content, that is, a time disposition process of the drug in the organism. The action of the drug on the organism is described by using pharmacodynamic (PD), i.e., a kinetic process in which the pesticide effects produced by the drug in vivo vary with time and concentration. The local and global PK/PD characteristics of the drug in vivo are described over time by integrating pharmacokinetics and pharmacodynamics so that the interaction between the drug and the organism can be more deeply expressed.

In an embodiment, FIG. 2 is a diagram illustrating a configuration of a physiological model structure according to an embodiment of the present application. As shown in FIG. 2, it is assumed that one organism includes N organs (which are organ 1, organ 2, organ 3 ... and organ N, respectively). The blood flow of each organ is associated with the venous blood flow and the arterial blood flow, that is, the drug in vivo process may be characterized by the association of blood flow in each organ with the venous blood flow and the arterial blood flow.

In S120, for each organism in a preset organism set, a physiological parameter, an anatomical parameter, and drug in vivo behavior data after the organism uses a specific drug are acquired as sample data.

In an embodiment, the physiological parameter and the anatomical parameter consist of a physiological characteristic parameter of a particular individual, the drug is described by using a physicochemical property, a structure characteristic and a pharmaceutical related parameter, and the drug in vivo behavior data is used for characterizing a local interaction between the drug and the organism or a complete in vivo behavior of the drug. In an embodiment, the physiological parameter and the anatomical parameter may include, but are not limited to, a body weight, a gender, an age, an enzyme activity, a transporter activity, and the like of each organism. The local interaction between the drug and the organism may be characterized by using the following parameters, but not limited to, the interaction with an enzyme, the interaction with a target, the concentration of the drug in each in vivo organ, and the binding rate of a protein. The complete in vivo behavior of the drug may be characterized by using the pharmacokinetic behavior.

In S130, a pre-created original prediction model is trained based on the sample data and the physiological model structure to obtain a corresponding target prediction model.

The original prediction model refers to a to-be-trained model framework created in advance. Exemplarily, the original prediction model may be a generative adversarial environment model. The generative adversarial environment model is an environment model trained by using adversarial training techniques for its network and learning of intelligent decision-making. In an embodiment, the physiological parameter and the anatomical parameter of each organism in the preset organism set and the drug in vivo behavior data obtained after each organism uses the specific drug are used as the sample data, and the sample data are input into the original prediction model based on the physiological model structure to perform an iterative training on the original prediction model until the prediction model obtained after the iterative training reaches the preset target, whereby the corresponding target prediction model may be obtained.

In the technical scheme of this embodiment, the physiological model structure for describing the drug in vivo process is established according to the physiological prior knowledge and the anatomical prior knowledge, the physiological parameter and the anatomical parameter of each organism in the preset organism set, and the drug in vivo behavior data of each organism after the specific drug is used are acquired as the sample data, and then the pre-created original prediction model is trained based on the sample data and the physiological model structure to obtain the target prediction model. In this manner, a data-driven model training process is achieved, and the drug in vivo behavior data are predicted based on the target prediction model, effectively improving the prediction accuracy of the model.

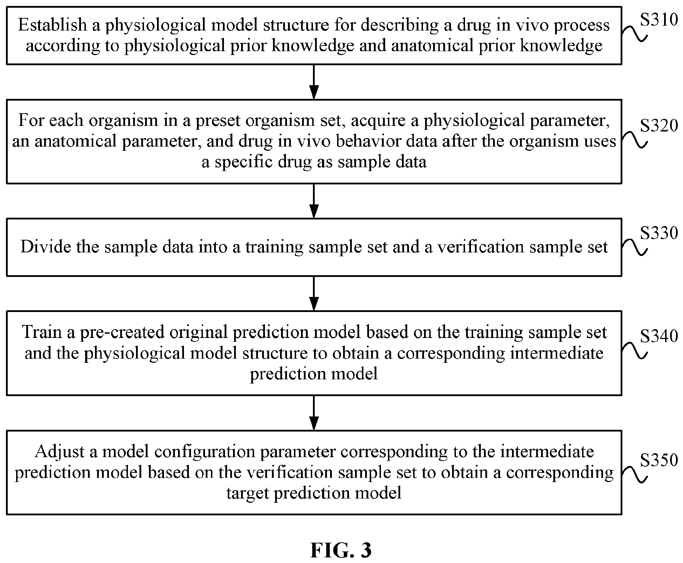

In an embodiment, FIG. 3 is a flowchart of another model construction method according to an embodiment of the present application. In this embodiment, an establishment process of the target prediction model is described on the basis of the above-described embodiment. As shown in FIG. 3, the method includes the following.

In S310, a physiological model structure for describing a drug in vivo process is established according to physiological prior knowledge and anatomical prior knowledge.

In an embodiment, S310 includes one of the following: a corresponding overall physiological model structure is established according to the physiological prior knowledge and the anatomical prior knowledge; or a corresponding local physiological model structure is established according to the physiological prior knowledge and the anatomical prior knowledge.

The overall physiological model structure is used for characterizing the complete in vivo behavior of the drug in one organism. The local physiological model structure is used for characterizing the local interaction between the drug and the organism. It should be understood that the corresponding overall or local physiological model structure may be established according to the physiological prior knowledge and the anatomical prior knowledge, and the establishment is performed according to an actual application requirement.

In S320, for each organism in a preset organism set, a physiological parameter, an anatomical parameter, and drug in vivo behavior data after the organism uses a specific drug are acquired as sample data.

In S330, the sample data are divided into a training sample set and a verification sample set.

In an embodiment, the training sample set refers to a set of training sample data for performing an iterative training on the original prediction model, and the verification sample set refers to a set of verification sample data for verifying the prediction model obtained by training. It should be understood that the training sample data in the training sample set are used in the training stage, and the verification sample data in the verification sample set are used in the verification stage. To ensure the prediction accuracy of the created target prediction model, the training sample data in the training sample set and the verification sample data in the verification sample set are configured to be mutually exclusive.

In S340, the pre-created original prediction model is trained based on the training sample set and the physiological model structure to obtain a corresponding intermediate prediction model.

In an embodiment, S340 includes S3401 and S3402.

In S3401, based on the physiological model structure, a drug-related parameter, a physiological parameter, and an anatomical parameter that are in the training sample set are input into the original prediction model to obtain corresponding first model generation drug in vivo behavior data by using a machine learning method according to first actual drug in vivo behavior data.

The first actual drug in vivo behavior data refers to drug in vivo behavior data that correspond to the drug-related parameter, the physiological parameter, and the anatomical parameter that are in the training sample set. It should be understood that the first actual drug in vivo behavior data refer to real drug in vivo behavior data obtained according to the drug-related parameter, the physiological parameter, and the anatomical parameter that are in the training sample set. The first model generation drug in vivo behavior data refer to drug in vivo behavior data generated by the original prediction model after the drug-related parameter, the physiological parameter, and the anatomical parameter of one organism are input into the original prediction model.

In an embodiment, the drug in vivo behavior data may be understood as timing data of in vivo behavior of the drug, and the timing data of the drug in vivo behavior may be modeled as a Markov decision process with a finite step size. The in vivo behavior data of the drug at each moment constitute a state space, a drug administration process is considered as an action, a transfer model is used to describe a state transfer of the drug in vivo behavior data at the current moment after the action is executed, and a modeling process is to obtain a state transfer model of the drug in vivo behavior data by data training. In each iteration of the model training stage, several pieces of corresponding drug in vivo behavior data may be generated according to the physiological parameter, the anatomical parameter and the drug-related parameter in the training sample set and used as the corresponding first model generation drug in vivo behavior data.

In S3402, an iterative training is performed on the original prediction model based on the first model generation drug in vivo behavior data and the first actual drug in vivo behavior data to obtain a corresponding intermediate prediction model.

The intermediate prediction model refers to a prediction model obtained by performing the iterative training on the original prediction model and using the first actual drug in vivo behavior data as the target. It should be understood that the difference between the first actual drug in vivo behavior data and the first model generation drug in vivo behavior data obtained by inputting the drug-related parameter, the physiological parameter, and the anatomical parameter of one organism into the intermediate prediction model is the smallest.

In S350, a model configuration parameter corresponding to the intermediate prediction model is adjusted based on the verification sample set to obtain the corresponding target prediction model.

**In** an embodiment, S350 includes S3501 to S3503.

In S3501, based on the physiological model structure, a drug-related parameter, a physiological parameter, and an anatomical parameter that are in the verification sample set are input into the intermediate prediction model to obtain corresponding second model generation drug in vivo behavior data.

The second model generation drug in vivo behavior data refer to drug in vivo behavior data generated by the intermediate prediction model after the drug-related parameter, the physiological parameter, and the anatomical parameter of one organism are input into the intermediate prediction model.

In S3502, a model configuration parameter of the intermediate prediction model is adjusted based on the second model generation drug in vivo behavior data and second actual drug in vivo behavior data in the verification sample set to obtain at least two corresponding candidate prediction models.

The second actual drug in vivo behavior data refer to drug in vivo behavior data corresponding to the drug-related parameter, the physiological parameter, and the anatomical parameter in the verification sample set. It should be understood that the second actual drug in vivo behavior data refer to real drug in vivo behavior data obtained according to the drug-related parameter, the physiological parameter, and the anatomical parameter that are in the verification sample set.

A candidate prediction model refers to a prediction model obtained by adjusting the model configuration parameter of the intermediate prediction model. It should be understood that each candidate prediction model corresponds to the intermediate prediction model having a different model configuration parameter. In an embodiment, the same group of drug-related parameters, physiological parameters, and anatomical parameters are input into the intermediate prediction model having different model configuration parameters to obtain multiple pieces of second model generation drug in vivo behavior data, and the intermediate prediction model having a model configuration parameter that corresponds to second model generation drug in vivo behavior data closest to the second actual drug in vivo behavior data and the intermediate prediction model having a model configuration parameter that corresponds to second model generation drug in vivo behavior data next close to the second actual drug in vivo behavior data are used as the corresponding candidate prediction models. Apparently, in a case of more than two candidate prediction models, a proximity degree between the second actual drug in vivo behavior data and the second model generation drug in vivo behavior data may be compared, the proximity degree is ranked in descending order, and then the top intermediate prediction models with model configuration parameters are used as the corresponding candidate prediction models.

In S3503, the corresponding target prediction model is determined according to a generalization performance of each candidate prediction model.

The generalization performance refers to the adaptability of each candidate prediction model to a fresh sample. In an embodiment, the generalization performance may be characterized by an evaluation indicator such as a generalization error and Akaike information quantity criterion. It should be understood that an optimal candidate prediction model with the minimum number of parameters may be selected according to the evaluation indicator such as the generalization error and Akaike information quantity criterion as the corresponding target prediction model.

In the technical scheme of this embodiment, based on the above-described embodiments, the iterative training is performed on the original prediction model based on the training sample set to obtain the corresponding intermediate prediction model, the model configuration parameter corresponding to the intermediate prediction model is adjusted based on the verification sample set to obtain the corresponding target prediction model, thereby implementing a data-driven model training process and improving the prediction accuracy of the drug in vivo behavior data.

In an embodiment, after the pre-created original prediction model is trained based on the sample data and the physiological model structure to obtain the corresponding target prediction model, the method further includes the following:
a target physiological parameter, a target anatomical parameter, and a target drug-related parameter that correspond to a target object are input into the target prediction model to obtain corresponding target model generation drug in vivo behavior data, where the target model generation drug in vivo behavior data include overall drug in vivo behavior data and an interaction characteristic of a target drug corresponding to the target drug-related parameter in an in vivo local organ; and
a model configuration parameter of the target prediction model is adjusted based on pre-acquired target actual drug in vivo behavior data and the target model generation drug in vivo behavior data to obtain a personalized prediction model corresponding to the target object

The target physiological parameter, the target anatomical parameter, the target drug-related parameter, the target model generation drug in vivo behavior data, and the target actual drug in vivo behavior data refer to related parameters matching the target object, and have similar explanations as the corresponding physiological parameter, the anatomical parameter and the drug-related parameter in the above-described embodiments, which is not described in detail herein.

In an embodiment, the target prediction model may be understood as one unified prediction model applicable to most organisms. In an actual operation process, the model configuration parameter of the target prediction model may be fine-tuned and optimized based on the target prediction model according to drug-related parameters, physiological parameters, and anatomical parameters of different organisms so that a personalized prediction model matching the corresponding organism is obtained.

In an embodiment, the target physiological parameter, the target anatomical parameter, and the target drug-related parameter of the target object may be input into the target prediction model based on the target prediction model to obtain the corresponding target model generation drug in vivo behavior data, and the model configuration parameter of the target prediction model is fine-tuned and optimized based on the target model generation drug in vivo behavior data and the target actual drug in vivo behavior data of the target object to obtain a personalized prediction model matching the target object.

In the technical scheme of this embodiment, based on the target prediction model, the model configuration parameter of the target prediction model may be fine-tuned and optimized according to the target physiological parameter, the target anatomical parameter, and the target drug-related parameter of the target object so that a corresponding personalized prediction model is obtained, thereby accurately predicting the local drug in vivo behavior data and the overall drug in vivo behavior data of the target object.

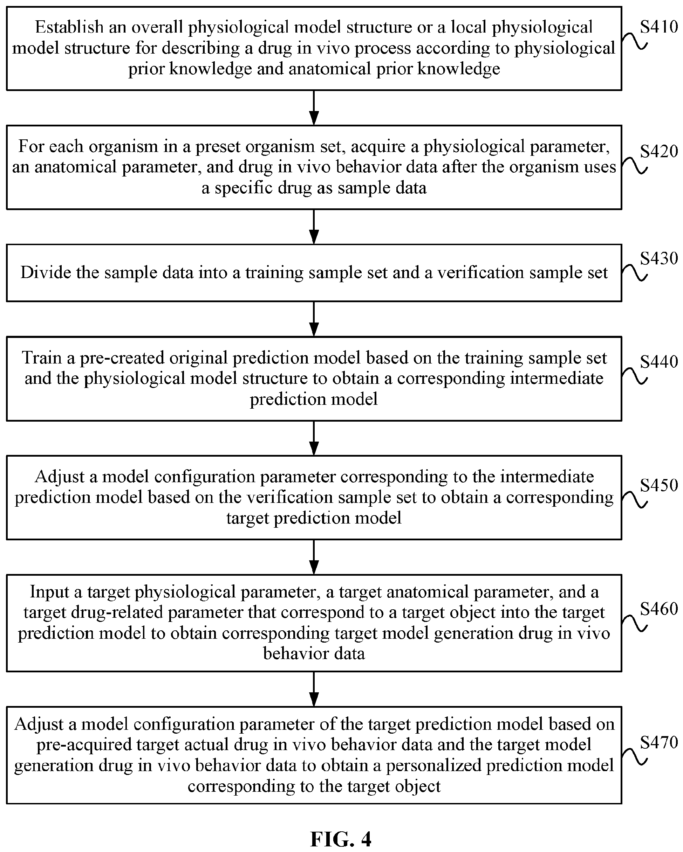

In an embodiment, FIG. 4 is a flowchart of another model construction method according to an embodiment of the present application. In this embodiment, the original prediction model being the generative adversarial environment model is used as an example, a model construction process will be described. The generative adversarial environment model includes a generator and a discriminator. As shown in FIG. 4, the model construction process in this embodiment includes the following steps.

In S410, an overall or local physiological model structure for describing a drug in vivo process is established according to physiological prior knowledge and anatomical prior knowledge.

In S420, for each organism in a preset organism set, a physiological parameter, an anatomical parameter, and drug in vivo behavior data after the organism uses a specific drug are acquired as sample data.

In S430, the sample data are divided into a training sample set and a verification sample set.

In S440, a pre-created original prediction model is trained based on the training sample set and the physiological model structure to obtain a corresponding intermediate prediction model.

In the present embodiment, the original prediction model is the generative adversarial environment model. The timing data of the drug in vivo behavior is modeled as a Markov decision process with a finite step size. The in vivo data of the drug at each moment constitute a state space, a drug administration process is considered as an action, a transfer model is used to describe a state transfer of the drug in vivo behavior data at the current moment after the action is executed, and a modeling process is to obtain a state transfer model of the drug in vivo behavior data obtained by data training. In each iteration of the model training session, the generator first generates several pieces of corresponding drug in vivo behavior data (that is, the first model generation drug in vivo behavior data) according to the physiological parameter, the anatomical parameter, and the drug-related parameter in the training sample set, and the update goal of the generator is to maximize the accumulated value of the prize given by the discriminator. The discriminator is configured to give a corresponding reward to the first model generation drug in vivo behavior data, and the update goal of the discriminator is to maximize the ability to distinguish between the first model generation drug in vivo behavior data and the first actual drug in vivo behavior data in the training sample set.

After the generator completes initialization, the discriminator maximizes its logarithmic estimation of the training sample data and minimizes its logarithmic estimation of the first model generation drug in vivo behavior data, and this process is similar to the calculation of the Jensen-Shannon divergence between the distribution of the first model generation drug in vivo behavior data and the distribution of the first actual drug in vivo behavior data. A calculation manner of a loss function corresponding to the optimization target of the discriminator is as follows: ${min}_{w} {E}_{\left(s,a,s′\right) ∼ {M}_{θ}} \left[log\left({D}_{w}\left(s,a,s′\right)\right)\right] + {E}_{\left(s,a,s′\right) ∼ B} \left[log\left(1-{D}_{w}\left(s,a,s′\right)\right)\right] .$

In the above-described formula, *(s, a, s'*) is state transfer data, *D*_{w} is the discriminator, w is a neural network parameter of the discriminator, *E(_{s,a,s' )~M_{θ}}* is an expectation of the state transfer data obtained by sampling from the current data, and ${min}_{w} {E}_{\left(s,a,s′\right) ∼ {M}_{θ}}$ is a value of w that minimizes the current loss function. Input of the generator is the state transfer data, output of the generator is a reward of the state transfer data, *log*(*D*_{w}(*s, a, s))* is a logarithm estimation of the output of the discriminator, *M*_{θ} is a transfer data set obtained by the generator, and B is a transfer data set in the training sample set. s refers to drug in vivo behavior data at this moment, *a* refers to a drug administration action or a dietary action at the stage of *s-s',* and s' refers to drug in vivo behavior data at the next moment.

The goal of the generator is to maximize the accumulated value of the reward function obtained by the generative adversarial environment model under the premise of a known strategy. Exemplarily, the process may use algorithms such as soft actor-critic (SAC) and proximal policy optimization (PPO). PPO is used as an example, this algorithm maximizes an advantage function between the updated model and the current model by using a gradient ascent method. Because the PPO uses the transfer data distribution of the current model to approximate the transfer data distribution of the updated model when calculating the optimization target of the environment transfer accumulated reward, it is required that the updated model after each iteration is closer to the transfer distribution of the current model. In this process, the accumulated reward is maximized while the distance between the updated model and the current model is limited, and a training result of each step has a monotonously increasing nature. After the PPO is used, the calculation method for the optimization target of the generation model is as follows: ${max}_{θ} {E}_{\left(s,a,s′\right) ∼ {M}_{{θ}_{old}}} \left[min\left\{\frac{{M}_{θ} \left(s,a,s′\right)}{{M}_{{θ}_{old}} \left(s,a,s′\right)}A\left(s,a,s′\right),clip\left(\frac{{M}_{θ} \left(s,a,s′\right)}{{M}_{{θ}_{old}} \left(s,a,s′\right)},1-ϵ,1+ϵ\right)A\left(s,a,s′\right)\right\}\right] .$

*M*_{θ*old*} is the current model, *M*_{θ} is a model updated at the current step, *A(s, a, s'*) is an advantage function in enhanced learning, and ε is generally about 0.6. To ensure that the distance between the model before update and the model after update is relatively small, the clip function ensures a small distance between the updated model and the current model.

In combination with the update of the discriminator and the update of the generator, when the discriminator achieves the theoretical optimization, the learning goal of the generative adversarial environment model may be equivalent to minimizing the distance between the distribution of the first model generation drug in vivo behavior data and the distribution of the first actual generation drug in vivo behavior data in the training sample set.

Compared with the supervised learning, the error term of the model with respect to the track length can be reduced from the square level to the linear level in the preceding scheme, thereby significantly improving the performance of the model. In conclusion, the optimization goal of learning of the generative adversarial environment model may be expressed as follows: ${max}_{θ} {min}_{w} {E}_{\left(s,a,s′\right) ∼ {M}_{θ}} \left[log\left({D}_{w}\left(s,a,s′\right)\right)\right] + {E}_{\left(s,a,s′\right) ∼ B} \left[log\left(1-{D}_{w}\left(s,a,s′\right)\right)\right] .$

In S450, a model configuration parameter corresponding to the intermediate prediction model is adjusted based on the verification sample set to obtain the corresponding target prediction model.

In the model verification stage, the generator generates several pieces of corresponding drug in vivo behavior data (that is, the second model generation drug in vivo behavior data in the above-described embodiments) according to the physiological parameter, the anatomical parameter, and the drug-related parameter in the verification sample set, and calculates the difference between the second model generation drug in vivo behavior data and the second actual drug in vivo behavior data in the verification sample set to evaluate the generalization performance of the model. On this basis, training and verification steps are continuously iterated to further optimize the model configuration parameter, and finally, an optimal model with the minimum number of parameters is selected according to the evaluation indicator such as the generalization error and Akaike information quantity criterion, where the generalization error is generally represented by using a mean absolute error (MAE): $MAE = \frac{1}{H} {\sum }_{l = 1}^{H} \left|\hat{{s}_{i}}-{s}_{i}\right| , \hat{{s}_{i}} ∼ {M}_{θ} , {s}_{i} ∼ B$

*H* represents the number of samples, $\hat{{s}_{i}}$ represents a predicted value of an *i*^{th} sample in the verification sample set *M*_{θ}, and *sᵢ* represents a real value of an *i*^{th} sample in the verification sample set B.

Akaike information criterion (AIC) is based on the concept of entropy, which can balance the complexity of the estimated model and the good performance of data fitting of this model, and AIC may be calculated by using the following formula. $AIC = \left(2k-2L\right) / n$

*k* is the number of parameters in the fitted model, *L* is a logarithmic likelihood value calculated by using the residual square sum, and *n* is the number of samples.

**In** S460, a target physiological parameter, a target anatomical parameter, and a target drug-related parameter that correspond to a target object are input into the target prediction model to obtain corresponding target model generation drug in vivo behavior data.

The target model generation drug in vivo behavior data include overall drug in vivo behavior data and an interaction characteristic of a target drug corresponding to the target drug-related parameter in an in vivo local organ.

**In** S470, a model configuration parameter of the target prediction model is adjusted based on pre-acquired target actual drug in vivo behavior data and the target model generation drug in vivo behavior data to obtain a personalized prediction model corresponding to the target object.

**In** the embodiment, interaction characteristics of the drug in other in vivo organs may be predicted according to partial actual measurement results (namely, the interaction characteristic of the target drug in a local in vivo organ) of the target object so that the overall drug in vivo behavior data of the drug may be restored.

**In** the technical scheme of this embodiment, the target prediction model is established by combining mechanistic prior knowledge such as known physiological parameters, anatomical parameters, pharmacokinetic and pharmacodynamics with various machine learning methods so that a data-driven model training process is implemented, the prediction accuracy of the drug in vivo behavior is high, and it may be used for the prediction of the drug in vivo behavior in terms of new drug preclinical development, clinical trial stage and clinical practice drug administration process, the basis for medicinal assessment, preclinical and clinical trial design, personalized drug administration are provided in the stages of drug discovery, development and application, and thus the development of drug development and clinical accurate drug administration can be greatly promoted.

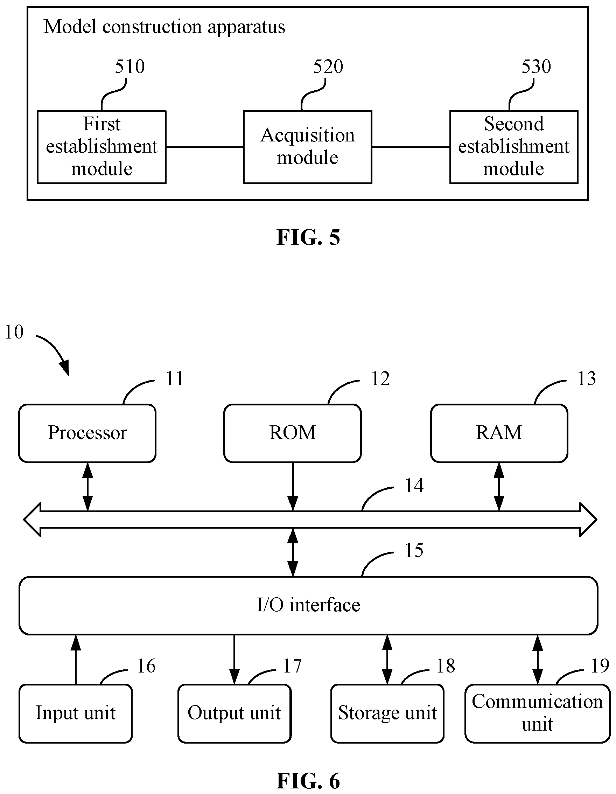

In an embodiment, FIG. 5 is a schematic structural diagram of a model construction apparatus according to an embodiment of the present application. As shown in FIG. 5, the apparatus includes a first establishment module 510, an acquisition module 520 and a second establishment module 530.

The first establishment module 510 is configured to establish a physiological model structure for describing a drug in vivo process according to physiological prior knowledge and anatomical prior knowledge.

The acquisition module 520 is configured to, for each organism in a preset organism set, acquire a physiological parameter, an anatomical parameter, and drug in vivo behavior data after the organism uses a specific drug as sample data.

The second establishment module 530 is configured to train a pre-created original prediction model based on the sample data and the physiological model structure to obtain a corresponding target prediction model.

In an embodiment, the first establishment module 510 is configured to establish a corresponding overall physiological model structure according to the physiological prior knowledge and the anatomical prior knowledge, or the first establishment module 510 is configured to establish a corresponding local physiological model structure according to the physiological prior knowledge and the anatomical prior knowledge.

In an embodiment, the physiological parameter and the anatomical parameter each consist of a physiological characteristic parameter of a specific individual, the drug is described by using a physicochemical property, a structure characteristic and a pharmaceutical related parameter, and the drug in vivo behavior data are used for characterizing a local interaction between the drug and the organism or a complete in vivo behavior of the drug.

In an embodiment, the second establishment module 530 includes a division unit, a training unit and an adjustment unit.

The division unit is configured to divide the sample data into a training sample set and a verification sample set.

The training unit is configured to train the pre-created original prediction model based on the training sample set and the physiological model structure to obtain a corresponding intermediate prediction model.

The adjustment unit is configured to adjust, based on the verification sample set, a model configuration parameter corresponding to the intermediate prediction model to obtain the corresponding target prediction model.

In an embodiment, the training unit includes a first generation subunit and a training subunit.

The first generation subunit is configured to input, based on the physiological model structure, a drug-related parameter, a physiological parameter, and an anatomical parameter that are in the training sample set into the original prediction model to obtain corresponding first model generation drug in vivo behavior data by using a machine learning method according to first actual drug in vivo behavior data.

The training subunit is configured to perform an iterative training on the original prediction model based on the first model generation drug in vivo behavior data and the first actual drug in vivo behavior data to obtain the corresponding intermediate prediction model.

In an embodiment, the adjustment unit includes a second generation subunit, an adjustment subunit and a determination subunit.

The second generation subunit is configured to input, based on the physiological model structure, a drug-related parameter, a physiological parameter, and an anatomical parameter that are in the verification sample set into the intermediate prediction model to obtain corresponding second model generation drug in vivo behavior data.

The adjustment subunit is configured to adjust the model configuration parameter of the intermediate prediction model based on the second model generation drug in vivo behavior data and second actual drug in vivo behavior data in the verification sample set to obtain at least two corresponding candidate prediction models.

The determination subunit is configured to determine the corresponding target prediction model according to a generalization performance of each candidate prediction model.

**In** an embodiment, after the pre-created original prediction model is trained based on the sample data and the physiological model structure to obtain the corresponding target prediction model, the model construction apparatus further includes a generation module and a third establishment module.

The generation module is configured to input a target physiological parameter, a target anatomical parameter, and a target drug-related parameter that correspond to a target object into the target prediction model to obtain corresponding target model generation drug in vivo behavior data. The target model generation drug in vivo behavior data include overall drug in vivo behavior data and an interaction characteristic of a target drug corresponding to the target drug-related parameter in an in vivo local organ.

The third establishment module is configured to adjust a model configuration parameter of the target prediction model based on pre-acquired target actual drug in vivo behavior data and the target model generation drug in vivo behavior data to obtain a personalized prediction model corresponding to the target object.

The model construction apparatus provided in the embodiment of the present application may execute the model construction method provided in any embodiment of the present application, and has corresponding function modules and beneficial effects for executing the method.

**In** an embodiment, FIG. 6 is a structural block diagram of an electronic device according to an embodiment of the present application. As shown in FIG. 6, FIG. 6 shows a schematic structural diagram of an electronic device 10 that may be used to implement the embodiments of the present application. The electronic device is intended to represent multiple forms of digital computers, such as a laptop computer, a desktop computer, a workbench, a personal digital assistant, a server, a blade server, a mainframe computer, and other suitable computers. The electronic device may further represent various mobile apparatuses, such as personal digital processing, a cellular phone, a smartphone, a wearable device (such as a helmet, glasses, and a watch), and other similar computing apparatuses. The components shown herein, their connections and relationships between these components, and the functions of these components, are illustrative merely and are not intended to limit implementations of the present application described and/or claimed herein.

As shown in FIG. 6, the electronic device 10 includes at least one processor 11 and a memory, such as a read-only memory (ROM) 12 and a random access memory (RAM) 13, communicatively connected to the at least one processor 11. The memory stores a computer program that may be executed by the at least one processor. The processor 11 may execute various proper actions and processes according to a computer program stored in the ROM 12 or a computer program loaded from a storage unit 18 to the RAM 13. In the RAM 13, various programs and data required for operations of the electronic device 10 may be further stored. The processor 11, the ROM 12 and the RAM 13 are connected to one another by using the bus 14. An input/output (I/O) interface 15 is also connected to the bus 14.

Multiple components in the electronic device 10 are connected to the I/O interface 15, and the multiple components include the following: an input unit 16 such as a keyboard and a mouse; an output unit 17 such as various types of displays and speakers; the storage unit 18 such as a magnetic disk or an optical disc; and a communications unit 19 such as a network adapter, a modem, and a wireless communications transceiver. The communications unit 19 allows the electronic device 10 to exchange information/data with other devices over a computer network such as the Internet and/or various telecommunications networks.

The processor 11 may be a variety of general-purpose and/or dedicated processing assemblies having processing and computing capabilities. Some examples of the processor 11 include, but are not limited to, a central processing unit (CPU), a graphics processing unit (GPU), a variety of special-purpose artificial intelligence (AI) computing chips, a variety of processors executing a machine learning model algorithm, a digital signal processor (DSP), any suitable processor, controller, microcontroller, and the like. The processor 11 performs the method and the processing described above, such as the model construction method.

In some embodiments, the model construction method may be implemented as the computer program, and the computer program is tangibly embodied in a computer-readable storage medium, such as the storage unit 18. In some embodiments, part or all of computer programs may be loaded and/or installed on the electronic device 10 via the ROM 12 and/or the communications unit 19. When the computer program is loaded into the RAM 13 and executed by the processor 11, one or more steps of the model construction method described above may be performed. Alternatively, in other embodiments, the processor 11 may be configured, in any other suitable manner (e.g., by means of firmware), to perform the model construction method.

Various implementations of the systems and technologies described above herein may be achieved in a digital electronic circuit system, an integrated circuit system, a field programmable gate array (FPGA), an application specific integrated circuit (ASIC), an application specific standard parts (ASSP), a system on chip (SOC), a complex programmable logic device (CPLD), a computer hardware, a firmware, a software, and/or combinations thereof. These implementations may include an implementation in one or more computer programs, and the one or more computer programs are executable and/or interpretable on a programmable system including at least one programmable processor, the programmable processor may be a special-purpose or general-purpose programmable processor for receiving data and instructions from a storage system, at least one input apparatus and at least one output apparatus and transmitting the data and instructions to the storage system, the at least one input apparatus and the at least one output apparatus.

Program codes for implementing the method of the present application may be written in any combination of one or more programming languages. These program codes may be provided for a general-purpose computer, a special-purpose computer, or a processor of another programmable data processing apparatus to enable the functions/operations specified in the flowchart and/or the block diagram to be implemented when the program codes are executed by the processor. The program codes may be executed entirely on a machine, partly on the machine, as a stand-alone software package, partly on the machine and partly on a remote machine, or entirely on the remote machine or server.

In the context of the present application, the computer-readable storage medium may be a tangible medium that may contain or store a computer program available for an instruction execution system, apparatus or device or a computer program used in conjunction with an instruction execution system, apparatus or device. The computer-readable storage medium may include, but is not limited to, an electronic, magnetic, optical, electromagnetic, infrared, or semiconductor system, apparatus, or device, or any appropriate combination of the foregoing. Alternatively, the computer-readable storage medium may be a machine-readable signal medium. More specific examples of the machine-readable storage medium may include an electrical connection based on one or more wires, a portable computer diskette, a hard disk, a RAM, a ROM, an erasable programmable read only memory (EPROM) or a flash memory, an optical fiber, a portable compact disc read-only memory (CD-ROM), an optical storage device, a magnetic storage device, or any appropriate combination of the foregoing.

To provide the interaction with the user, the system and technology described here may be implemented on the electronic device. The electronic device has a display device (such as a cathode ray tube (CRT) or liquid crystal display (LCD) monitor) for displaying information to the user; and a keyboard and a pointing apparatus (such as a mouse or a trackball) through which the user may provide input to the electronic device. Other kinds of apparatuses may also be configured to provide the interaction for the user; for example, feedback provided to the user may be sensory feedback in any form (such as visual feedback, auditory feedback, or haptic feedback); and input from the user may be received in any form (including acoustic input, speech input, or haptic input).

The system and technology described here may be implemented in a calculation system including a back-end component (such as a data server), or a calculation system including a middleware component (such as an application server), or a calculation system including a front-end component (such as a client computer having a graphical user interface or a web browser through which the user may interact with the implementations of the system and technology described herein), or a calculation system including any combination of such back-end component, middleware component, or front-end component. The components of the system may be interconnected by any form or medium of digital data communication (such as a communication network). Examples of the communication network include a local area network (LAN), a wide area network (WAN), a blockchain network and the Internet.

A computer system may include a client and a server. The client and the server are generally facing away from each other and typically interact through the communication network. A relationship between the client and the server arises by virtue of computer programs running on respective computers and having a client-server relationship with each other. The server may be a cloud server, also referred to as a cloud calculation server or a cloud host. As a host product in a cloud calculation service system, the server solves the defects of difficult management and weak service scalability in a traditional physical host and a virtual private server (VPS) service.

It should be understood that various forms of the flows shown above, reordering, adding or deleting steps may be used. For example, as long as the desired result of the technical scheme disclosed in the present application may be achieved, the steps recited in the present application may be executed in parallel, sequentially or in different orders, which is not limited herein.

The above-described implementations are not to be construed as limiting the scope of protection of the present application.
execution system, apparatus or device or a computer program used in conjunction with an instruction execution system, apparatus or device. The computer-readable storage medium may include, but is not limited to, an electronic, magnetic, optical, electromagnetic, infrared, or semiconductor system, apparatus, or device, or any appropriate combination of the foregoing. Alternatively, the computer-readable storage medium may be a machine-readable signal medium. More specific examples of the machine-readable storage medium may include an electrical connection based on one or more wires, a portable computer diskette, a hard disk, a RAM, a ROM, an erasable programmable read only memory (EPROM) or a flash memory, an optical fiber, a portable compact disc read-only memory (CD-ROM), an optical storage device, a magnetic storage device, or any appropriate combination of the foregoing.

To provide the interaction with the user, the system and technology described here may be implemented on the electronic device. The electronic device has a display device (such as a cathode ray tube (CRT) or liquid crystal display (LCD) monitor) for displaying information to the user; and a keyboard and a pointing apparatus (such as a mouse or a trackball) through which the user may provide input to the electronic device. Other kinds of apparatuses may also be configured to provide the interaction for the user; for example, feedback provided to the user may be sensory feedback in any form (such as visual feedback, auditory feedback, or haptic feedback); and input from the user may be received in any form (including acoustic input, speech input, or haptic input).

The system and technology described here may be implemented in a calculation system including a back-end component (such as a data server), or a calculation system including a middleware component (such as an application server), or a calculation system including a front-end component (such as a client computer having a graphical user interface or a web browser through which the user may interact with the implementations of the system and technology described herein), or a calculation system including any combination of such back-end component, middleware component, or front-end component. The components of the system may be interconnected by any form or medium of digital data communication (such as a communication network). Examples of the communication network include a local area network (LAN), a wide area network (WAN), a blockchain network and the Internet.

A computer system may include a client and a server. The client and the server are generally facing away from each other and typically interact through the communication network. A relationship between the client and the server arises by virtue of computer programs running on respective computers and having a client-server relationship with each other. The server may be a cloud server, also referred to as a cloud calculation server or a cloud host. As a host product in a cloud calculation service system, the server solves the defects of difficult management and weak service scalability in a traditional physical host and a virtual private server (VPS) service.

It should be understood that various forms of the flows shown above, reordering, adding or deleting steps may be used. For example, as long as the desired result of the technical scheme disclosed in the present application may be achieved, the steps recited in the present application may be executed in parallel, sequentially or in different orders, which is not limited herein.

The above-described implementations are not to be construed as limiting the scope of protection of the present application. The scope of the invention is limited by the scope of the appended claims.

## Claims

1. A computer-implemented model construction method, performed by an electronic device, comprising:
establishing (S110, S310) a physiological model structure for describing a drug in vivo process according to physiological prior knowledge and anatomical prior knowledge, wherein the physiological model structure is configured to characterize an association of a local interaction between the drug and an organism with physiological characteristics, anatomical characteristics and drug characteristics, the local interaction comprises an action of the organism on the drug and an action of the drug on the organism, the action of the organism on the drug is used to characterize local and global pharmacokinetic characteristics of the drug in vivo process over time, and the action of the drug on the organism is used to characterize local and global pharmacodynamic characteristics of the drug in vivo process over time;
for each organism in a preset organism set, acquiring (S120, S320, S420) a physiological parameter, an anatomical parameter, and drug in vivo behavior data after the respective organism uses a specific drug as sample data; and
training (S130) a pre-created original prediction model based on the sample data and the physiological model structure to obtain a corresponding target prediction model;
wherein training (S130) the pre-created original prediction model based on the sample data and the physiological model structure to obtain the corresponding target prediction model comprises:
dividing (S330, S430) the sample data into a training sample set and a verification sample set;
training (S340, S440) the pre-created original prediction model based on the training sample set and the physiological model structure to obtain a corresponding intermediate prediction model; and
adjusting (S350, S450), based on the verification sample set, a model configuration parameter corresponding to the intermediate prediction model to obtain the corresponding target prediction model; and
wherein adjusting, based on the verification sample set, the model configuration parameter corresponding to the intermediate prediction model to obtain the corresponding target prediction model comprises:
inputting, based on the physiological model structure, a drug-related parameter, a physiological parameter, and an anatomical parameter that are in the verification sample set into the intermediate prediction model to obtain corresponding second model generation drug in vivo behavior data;
adjusting the model configuration parameter of the intermediate prediction model based on the second model generation drug in vivo behavior data and second actual drug in vivo behavior data in the verification sample set to obtain at least two corresponding candidate prediction models; and
determining the corresponding target prediction model according to a generalization performance of each of the at least two candidate prediction models.

2. The method of claim 1, wherein establishing (S110, S310) the physiological model structure for describing the drug in vivo process according to the physiological prior knowledge and the anatomical prior knowledge comprises:
establishing a corresponding overall physiological model structure according to the physiological prior knowledge and the anatomical prior knowledge; or
establishing a corresponding local physiological model structure according to the physiological prior knowledge and the anatomical prior knowledge.

3. The method of claim 1, wherein the physiological parameter and the anatomical parameter each consist of a physiological characteristic parameter of a specific individual, the drug is described by using a physicochemical property, a structure characteristic and a pharmaceutical related parameter, and the drug in vivo behavior data are used for characterizing a local interaction between the drug and the organism or a complete in vivo behavior of the drug.

4. The method of claim 1, wherein training the pre-created original prediction model based on the training sample set and the physiological model structure to obtain the corresponding intermediate prediction model comprises:
inputting, based on the physiological model structure, a drug-related parameter, a physiological parameter, and an anatomical parameter that are in the training sample set into the original prediction model to obtain corresponding first model generation drug in vivo behavior data according to first actual drug in vivo behavior data by using a machine learning method; and
performing an iterative training on the original prediction model based on the first model generation drug in vivo behavior data and the first actual drug in vivo behavior data to obtain the corresponding intermediate prediction model.

5. The method of any one of claims 1 to 4, wherein after training (S130) the pre-created original prediction model based on the sample data and the physiological model structure to obtain the corresponding target prediction model, the method further comprises:
inputting (S460) a target physiological parameter, a target anatomical parameter, and a target drug-related parameter that correspond to a target object into the target prediction model to obtain corresponding target model generation drug in vivo behavior data, wherein the target model generation drug in vivo behavior data comprise overall drug in vivo behavior data and an interaction characteristic of a target drug corresponding to the target drug-related parameter in an in vivo local organ; and
adjusting (S470) a model configuration parameter of the target prediction model based on pre-acquired target actual drug in vivo behavior data and the target model generation drug in vivo behavior data to obtain a personalized prediction model corresponding to the target object.

6. An electronic device, comprising:
at least one processor; and
a memory communicatively connected to the at least one processor,
wherein the memory stores a computer program executable by the at least one processor, and the computer program, when executed by the at least one processor, causes the at least one processor to perform the model construction method of any one of claims 1 to 5.

7. A computer-readable storage medium storing a computer instruction, wherein the computer instruction is configured to, when executed by a processor, implement the model construction method of any one of claims 1 to 5.

## Patentansprüche

1. Computerimplementiertes Modellkonstruktionsverfahren, durchgeführt durch eine elektronische Vorrichtung, umfassend:
Festlegen (S110, S310) einer Struktur eines physiologischen Modells zur Beschreibung eines Arzneimittel-in-vivo-Prozesses gemäß bereits vorhandener physiologischer Kenntnisse und bereits vorhandener anatomischer Kenntnisse, wobei die Struktur des physiologischen Modells ausgelegt ist, um eine Verknüpfung einer lokalen Interaktion zwischen dem Arzneimittel und einem Organismus mit physiologischen Merkmalen, anatomischen Merkmalen und Arzneimittelmerkmalen zu charakterisieren, wobei die lokale Interaktion eine Wirkung des Organismus auf das Arzneimittel und eine Wirkung des Arzneimittels auf den Organismus umfasst, wobei die Wirkung des Organismus auf das Arzneimittel verwendet wird, um lokale und globale pharmakokinetische Merkmale des Arzneimittel-in-vivo-Prozesses im Lauf der Zeit zu charakterisieren, und die Wirkung des Arzneimittels auf den Organismus verwendet wird, um lokale und globale pharmakodynamische Merkmale des Arzneimittel-in-vivo-Prozesses im Lauf der Zeit zu charakterisieren;
für jeden Organismus in einer vorgegebenen Menge von Organismen Erfassen (S120, S320, S420) eines physiologischen Parameters, eines anatomischen Parameters und von Arzneimittel-in-vivo-Verhaltensdaten nach der Verwendung eines spezifischen Arzneimittels durch den jeweiligen Organismus als Probendaten, und
Trainieren (S130) eines im Vorfeld erstellten originalen Vorhersagemodells basierend auf den Probendaten und der Struktur des physiologischen Modells, um ein entsprechendes Zielvorhersagemodell zu erhalten,
wobei das Trainieren (S130) des im Vorfeld erstellten originalen Vorhersagemodells basierend auf den Probendaten und der Struktur des physiologischen Modells, um das entsprechende Zielvorhersagemodell zu erhalten, Folgendes umfasst:
Aufteilen (S330, S430) der Probendaten in eine Trainingsprobenmenge und eine Nachweisprobenmenge;
Trainieren (S340, S440) des im Vorfeld erstellten originalen Vorhersagemodells basierend auf der Trainingsprobenmenge und der Struktur des physiologischen Modells, um ein entsprechendes Zwischenvorhersagemodell zu erhalten, und
Anpassen (S350, S450) eines dem Zwischenvorhersagemodell entsprechenden Modellkonfigurationsparameters basierend auf der Nachweisprobenmenge, um das entsprechende Zielvorhersagemodell zu erhalten, und
wobei das Anpassen des dem Zwischenvorhersagemodell entsprechenden Modellkonfigurationsparameters basierend auf der Nachweisprobenmenge, um das entsprechende Zielvorhersagemodell zu erhalten, Folgendes umfasst:
Eingeben eines arzneimittelbezogenen Parameters, eines physiologischen Parameters und eines anatomischen Parameters, die sich in der Nachweisprobenmenge befinden, basierend auf der Struktur des physiologischen Modells in das Zwischenvorhersagemodell, um entsprechende zweite durch Modellgenerierung erzeugte Arzneimittel-in-vivo-Verhaltensdaten zu erhalten;
Anpassen des Modellkonfigurationsparameters des Zwischenvorhersagemodells basierend auf den zweiten durch Modellgenerierung erzeugten Arzneimittel-in-vivo-Verhaltensdaten und zweiten tatsächlichen Arzneimittel-in-vivo-Verhaltensdaten in der Nachweisprobenmenge, um mindestens zwei Vorhersagemodellkandidaten zu erhalten, und
Bestimmen des entsprechenden Zielvorhersagemodells gemäß einer Generalisierungsleistung jedes der mindestens zwei Vorhersagemodellkandidaten.

2. Verfahren nach Anspruch 1, wobei das Festlegen (S110, S310) der Struktur des physiologischen Modells zur Beschreibung des Arzneimittel-in-vivo-Prozesses gemäß den bereits bekannten physiologischen Kenntnissen und den bereits bekannten anatomischen Kenntnissen Folgendes umfasst:
Festlegen einer entsprechenden Gesamtstruktur des physiologischen Modells gemäß den bereits bekannten physiologischen Kenntnissen und den bereits bekannten anatomischen Kenntnissen oder
Festlegen einer entsprechenden lokalen Struktur des physiologischen Modells gemäß den bereits bekannten physiologischen Kenntnissen und den bereits bekannten anatomischen Kenntnissen.

3. Verfahren nach Anspruch 1, wobei der physiologische Parameter und der anatomische Parameter jeweils aus einem physiologischen charakteristischen Parameter eines spezifischen Individuums bestehen, das Arzneimittel mithilfe einer physikochemischen Eigenschaft, einer Struktureigenschaft und eines pharmazeutikabezogenen Parameters beschrieben wird und die Arzneimittel-in-vivo-Verhaltensdaten zur Charakterisierung einer lokalen Interaktion zwischen dem Arzneimittel und dem Organismus oder eines vollständigen In-vivo-Verhaltens des Arzneimittels verwendet werden.

4. Verfahren nach Anspruch 1, wobei das Trainieren des im Vorfeld erstellten originalen Vorhersagemodells basierend auf der Trainingsprobenmenge und der Struktur des physiologischen Modells, um das entsprechende Zwischenvorhersagemodell zu erhalten, Folgendes umfasst:
Eingeben eines arzneimittelbezogenen Parameters, eines physiologischen Parameters und eines anatomischen Parameters, die sich in der Trainingsprobenmenge befinden, basierend auf der Struktur des physiologischen Modells in das originale Vorhersagemodell, um entsprechende erste durch Modellgenerierung erzeugte Arzneimittel-in-vivo-Verhaltensdaten gemäß ersten tatsächlichen Arzneimittel-in-vivo-Verhaltensdaten mithilfe eines Verfahrens des maschinellen Lernens zu erhalten, und
Durchführen eines iterativen Trainings an dem originalen Vorhersagemodell basierend auf den ersten durch Modellgenerierung erzeugten Arzneimittel-in-vivo-Verhaltensdaten und den ersten tatsächlichen Arzneimittel-in-vivo-Verhaltensdaten, um das entsprechende Zwischenvorhersagemodell zu erhalten.

5. Verfahren nach einem der Ansprüche 1 bis 4, wobei das Verfahren nach dem Trainieren (S130) des im Vorfeld erstellten originalen Vorhersagemodells basierend auf den Probendaten und der Struktur des physiologischen Modells, um das entsprechende Zielvorhersagemodell zu erhalten, ferner Folgendes umfasst:
Eingeben (S460) eines physiologischen Zielparameters, eines anatomischen Zielparameters und eines arzneimittelbezogenen Zielparameters, die einem Zielobjekt entsprechen, in das Zielvorhersagemodell, um entsprechende durch Modellgenerierung erzeugte Arzneimittel-in-vivo-Zielverhaltensdaten zu erhalten, wobei die durch Modellgenerierung erzeugten Arzneimittel-in-vivo-Zielverhaltensdaten Arzneimittel-in-vivo-Gesamtverhaltensdaten und ein Interaktionsmerkmal eines Zielarzneimittels entsprechend dem arzneimittelbezogenen Zielparameter in einem lokalen in-vivo-Organ umfassen, und
Anpassen (S470) eines Modellkonfigurationsparameters des Zielvorhersagemodells basierend auf im Vorfeld erfassten tatsächlichen Arzneimittel-in-vivo-Zielverhaltensdaten und den durch Modellgenerierung erzeugten Arzneimittel-in-vivo-Zielverhaltensdaten, um ein personalisiertes Vorhersagemodell zu erhalten, das dem Zielobjekt entspricht.

6. Elektronische Vorrichtung, umfassend:
mindestens einen Prozessor und
einen Speicher, der kommunikativ mit dem mindestens einen Prozessor verbunden ist,
wobei der Speicher ein Computerprogramm speichert, das durch den mindestens einen Prozessor ausführbar ist, und das Computerprogramm bei Ausführung durch den mindestens einen Prozessor bewirkt, dass der mindestens eine Prozessor das Modellkonstruktionsverfahren nach einem der Ansprüche 1 bis 5 durchführt.

7. Computerlesbares Speichermedium, das eine Computeranweisung speichert, wobei die Computeranweisung ausgelegt ist, um bei Ausführung durch einen Prozessor das Modellkonstruktionsverfahren nach einem der Ansprüche 1 bis 5 zu implementieren.

## Revendications

1. Procédé de construction de modèle mis en œuvre par ordinateur, réalisé par un dispositif électronique, comprenant :
l'établissement (S110, S310) d'une structure de modèle physiologique pour la description d'un processus in vivo de médicament en fonction de connaissances physiologiques précédentes et de connaissances anatomiques précédentes, dans lequel la structure de modèle physiologique est configurée pour caractériser une association d'une interaction locale entre le médicament et un organisme avec des caractéristiques physiologiques, des caractéristiques anatomiques et des caractéristiques de médicament, l'interaction locale comprend une action de l'organisme sur le médicament et une action du médicament sur l'organisme, l'action de l'organisme sur le médicament est utilisée pour caractériser des caractéristiques pharmacocinétiques locales et globales du médicament dans le processus in vivo au cours du temps, et l'action du médicament sur l'organisme est utilisée pour caractériser des caractéristiques pharmacodynamiques locales et globales du médicament dans le processus in vivo au cours du temps ;
pour chaque organisme dans un ensemble d'organismes prédéfini, l'acquisition (S120, S320, S420) d'un paramètre physiologique, d'un paramètre anatomique et de données de comportement in vivo de médicament après que l'organisme respectif a utilisé un médicament spécifique comme données échantillons ; et
l'entraînement (S130) d'un modèle de prédiction d'origine pré-créé sur la base des données échantillons et de la structure de modèle physiologique pour obtenir un modèle de prédiction cible correspondant ;
dans lequel l'entraînement (S130) du modèle de prédiction d'origine pré-créé sur la base des données échantillons et de la structure de modèle physiologique pour obtenir le modèle de prédiction cible correspondant comprend :
la division (S330, S430) des données échantillons en un ensemble d'échantillons d'entraînement et un ensemble d'échantillons de vérification ;
l'entraînement (S340, S440) du modèle de prédiction d'origine pré-créé sur la base de l'ensemble d'échantillons d'entraînement et de la structure de modèle physiologique pour obtenir un modèle de prédiction intermédiaire correspondant ; et
l'ajustement (S350, S450), sur la base de l'ensemble d'échantillons de vérification, d'un paramètre de configuration de modèle correspondant au modèle de prédiction intermédiaire pour obtenir le modèle de prédiction cible correspondant ; et
dans lequel l'ajustement, sur la base de l'ensemble d'échantillons de vérification, du paramètre de configuration de modèle correspondant au modèle de prédiction intermédiaire pour obtenir le modèle de prédiction cible correspondant comprend :
l'entrée, sur la base de la structure de modèle physiologique, d'un paramètre lié au médicament, d'un paramètre physiologique et d'un paramètre anatomique qui se trouvent dans l'ensemble d'échantillons de vérification dans le modèle de prédiction intermédiaire pour obtenir des deuxièmes données de génération de comportement in vivo de médicament de modèle correspondantes ;
l'ajustement du paramètre de configuration de modèle du modèle de prédiction intermédiaire sur la base des deuxièmes données de génération de comportement in vivo de médicament de modèle et des deuxièmes données réelles de comportement in vivo de médicament dans l'ensemble d'échantillons de vérification pour obtenir au moins deux modèles de prédiction candidats correspondants ; et
la détermination du modèle de prédiction cible correspondant en fonction d'une performance de généralisation de chacun des au moins deux modèles de prédiction candidats.

2. Procédé selon la revendication 1, dans lequel l'établissement (S110, S310) de la structure de modèle physiologique pour la description du processus in vivo de médicament en fonction des connaissances physiologiques précédentes et des connaissances anatomiques précédentes comprend :
l'établissement d'une structure de modèle physiologique globale correspondante en fonction des connaissances physiologiques précédentes et des connaissances anatomiques précédentes ; ou
l'établissement d'une structure de modèle physiologique local correspondante en fonction des connaissances physiologiques précédentes et des connaissances anatomiques précédentes.

3. Procédé selon la revendication 1, dans lequel chacun du paramètre physiologique et du paramètre anatomique est un paramètre de caractéristique physiologique d'un individu spécifique, le médicament est décrit en utilisant une propriété physico-chimique, une caractéristique de structure et un paramètre lié à un produit pharmaceutique, et les données de comportement in vivo de médicament sont utilisées pour caractériser une interaction locale entre le médicament et l'organisme ou un comportement in vivo complet du médicament.

4. Procédé selon la revendication 1, dans lequel l'entraînement du modèle de prédiction d'origine pré-créé sur la base de l'ensemble d'échantillons d'entraînement et de la structure de modèle physiologique pour obtenir le modèle de prédiction intermédiaire correspondant comprend :
l'entrée, sur la base de la structure de modèle physiologique, d'un paramètre lié au médicament, d'un paramètre physiologique et d'un paramètre anatomique qui se trouvent dans l'ensemble d'échantillons d'entraînement dans le modèle de prédiction d'origine pour obtenir des premières données de génération de comportement in vivo de médicament de modèle correspondantes selon des premières données réelles de comportement in vivo de médicament en utilisant un procédé d'apprentissage automatique ; et
la réalisation d'un entraînement itératif sur le modèle de prédiction d'origine sur la base des premières données de génération de comportement in vivo de médicament de modèle et des premières données réelles de comportement in vivo de médicament pour obtenir le modèle de prédiction intermédiaire correspondant.

5. Procédé selon l'une quelconque des revendications 1 à 4, dans lequel, après l'entraînement (S130) du modèle de prédiction d'origine pré-créé sur la base des données échantillons et de la structure de modèle physiologique pour obtenir le modèle de prédiction cible correspondant, le procédé comprend en outre :
l'entrée (S460) d'un paramètre physiologique cible, d'un paramètre anatomique cible et d'un paramètre lié au médicament cible qui correspondent à un objet cible dans le modèle de prédiction cible pour obtenir des données correspondantes de comportement in vivo de médicament de génération de modèle cible, dans lequel les données de génération de comportement in vivo de médicament de modèle cible comprennent des données globales de comportement in vivo de médicament et une caractéristique d'interaction d'un médicament cible correspondant au paramètre lié au médicament cible dans un organe local in vivo ; et
l'ajustement (S470) d'un paramètre de configuration de modèle du modèle de prédiction cible sur la base de données réelles de comportement in vivo de médicament cible pré-acquises et des données de génération de comportement in vivo de médicament de modèle cible pour obtenir un modèle de prédiction personnalisé correspondant à l'objet cible.

6. Dispositif électronique, comprenant :
au moins un processeur ; et
une mémoire reliée en communication à l'au moins un processeur,
dans lequel la mémoire stocke un programme informatique exécutable par l'au moins un processeur, et le programme informatique, lorsqu'il est exécuté par l'au moins un processeur, amène l'au moins un processeur à réaliser le procédé de construction de modèle selon l'une quelconque des revendications 1 à 5.

7. Support de stockage lisible par ordinateur stockant une instruction informatique, dans lequel l'instruction informatique est configurée, lorsqu'elle est exécutée par un processeur, pour mettre en œuvre le procédé de construction de modèle selon l'une quelconque des revendications 1 à 5.
